# EUROPÄISCHE PATENTSCHRIFT

(11) **EP 3 210 537 B1**
(45) Veröffentlichungstag und Bekanntmachung des Hinweises auf die Patenterteilung: **22.08.2018**
(21) Anmeldenummer: 17170648.4
(22) Anmeldetag: 11.05.2017
(51) Int. Cl.: A61B 6/00, A61B 6/08, A61B 6/02, A61B 6/04

(54) **ERSTELLUNG EINES DREIDIMENSIONALEN ABBILDS EINES KÖRPERTEILS DURCH EIN RÖNTGENGERÄT**
CREATION OF A THREE DIMENSIONAL IMAGE OF A PORTION OF A BODY BY A X-RAY APPARATUS
FABRICATION D'UNE IMAGE TRIDIMENSIONNELLE D'UNE PARTIE DE CORPS AU MOYEN D'UN APPAREIL À RAYONS X

(30) Priorität: 07.06.2016 DE 102016210003
(43) Veröffentlichungstag der Anmeldung: 30.08.2017
(73) Patentinhaber: Siemens Healthcare GmbH, 91052 Erlangen (DE)
(72) Erfinder: Redel, Thomas, 91099 Poxdorf (DE); Scheuering, Michael, 90425 Nürnberg (DE)

(56) Entgegenhaltungen:
- US-A1- 2008 171 936
- US-A1- 2013 266 123
- SHENGXIAN TU ET AL: "In vivo assessment of bifurcation optimal viewing angles and bifurcation angles by three-dimensional (3D) quantitative coronary angiography", THE INTERNATIONAL JOURNAL OF CARDIAC IMAGING, KLUWER ACADEMIC PUBLISHERS, DO, Bd. 28, Nr. 7, 15. Dezember 2011 (2011-12-15), Seiten 1617-1625, XP035125848, ISSN: 1573-0743, DOI: 10.1007/S10554-011-9996-X

## Beschreibung

Die Erfindung betrifft ein Verfahren zum Betreiben eines Röntgengeräts für ein Erstellen eines dreidimensionalen Abbilds eines Körperteils. Sie betrifft ebenfalls ein Röntgengerät zum Aufnehmen von zumindest zwei Röntgenbildern für ein Erstellen eines dreidimensionalen Abbilds eines Körperteils mit einer Aufnahmeeinheit zum Aufnehmen von Röntgenaufnahmen aus an dem Röntgengerät einstellbaren und durch die Aufnahmeeinheit unter manueller Kontrolle anfahrbaren Aufnahmewinkeln.

Zur Berechnung eines Wertes einer fraktionellen Flussreserve (FFR) wird oft auf ein dreidimensionales Modell eines Körperteiles, insbesondere eines Blutgefäßes und/oder eines Blutgefäßteils und/oder eines Organs zurückgegriffen, welches mittels einer Angiographie, also unter Verwendung von zwei unter einem verschiedenen Winkel aufgenommenen Röntgenaufnahmen erstellt wird. Eine Aufnahme oder Röntgenaufnahme wird hier auch als Akquisition oder (Angiographie-)Szene beschrieben. Die in einer Aufnahme enthaltenen Daten repräsentieren einen Film mit einer Vielzahl von Einzelbildern unter einer im Allgemeinen fest beziehungsweise unveränderlich vorgegebenen Angulation, das heißt einem relativ zu dem Körperteil für die Akquisition vorgegebenen Aufnahmewinkel.

Das Modell wird nachträglich aus zwei Aufnahmen oder Akquisitionen erstellt. Dafür wird je ein geeignetes Einzelbild der beiden Aufnahmen ausgewählt. Aus diesen wird von einem Rechner auf die dreidimensionale Struktur des Körperteils rückgeschlossen und ein dreidimensionales Abbild des Körperteils als 3D-Modell erstellt. Daraufhin kann der Körperteil im Rahmen der Quantifizierung der fraktionellen Flussreserve analysiert werden. Ein resultierender Wert als Maßzahl für ein Diagnoseergebnis des Körperteils wird auch als angioFFR-Wert bezeichnet. Typischerweise werden dabei für eine diagnostische Befundung zwischen 9 und 20 Röntgenaufnahmen aufgenommen. Die Röntgenaufnahmen werden oft auch für weitere Verwendungszwecke aufgenommen, was dazu führt, dass für die Modellerstellung eventuell Angiographieszenen oder Röntgenaufnahmen verwendet werden oder verwendet werden müssen, welcher für eine andere Verwendung optimiert sind. Das führt zu einer eingeschränkten Qualität des 3D-Modells des Körperteils.

Alternativ können für ein im Hinblick auf die Auswertung im Rahmen einer FFR-Analyse optimiertes dreidimensionales Abbild nachträglich nochmals eine oder mehrere Aufnahmen gemacht werden. Dies stellt für den Patienten bzw. dessen Körperteil eine gesundheitliche Belastung dar. Die nachträglich gemachte Röntgenaufnahme beziehungsweise die Auswahl der bereits gewonnenen Röntgenaufnahmen für die Erstellung des 3D-Modells oder des dreidimensionalen Abbilds des Körperteils basiert hier heute wesentlich auf der individuellen Erfahrung der Bedienperson des Röntgengerätes, typischerweise eines Arztes.

Typischerweise werden also im Stand der Technik zunächst die beiden Röntgenaufnahmen aufgenommen, sodann gespeichert und nachträglich aus den gegebenen und somit nicht mehr beeinflussbaren Röntgenaufnahmen ein dreidimensionales Modell erstellt.

Ein weiterer großer Nachteil in bestehenden Systemen und Verfahren ist, dass oft Röntgenaufnahmen abgespeichert werden, welche bereits im Hinblick auf eine visuelle Betrachtung optimiert, das heißt nachbearbeitet wurden. Die Folge ist, dass der angioFFR-Wert nachträglich nur mit einer verminderten Qualität bestimmt werden kann. Dies kann zum Beispiel darin begründet sein, dass die für eine visuelle Betrachtung vorteilhaften Bearbeitungsschritte eine nachträgliche andersartige Bearbeitung erschweren, wie sie zum Beispiel für eine densiometrische Auswertung erforderlich ist.

Die US 2008 / 0 171 936 A1 befasst sich mit einer Vorrichtung und einer Methode zum Beobachten eines medizinischen Instruments in einem Körpervolumen eines Patienten. Dabei werden eine 3D-Repräsentation eines Bewegungskorridors in dem Körpervolumen und eine aktuelle Projektion bestimmt. Davon ausgehend wird eine aktuelle Position des Instruments bezogen auf die 3D-Repräsentation bestimmt. Anschließend wird bezogen auf die aktuelle Position des Instruments eine optimale Projektionsgeometrie bestimmt und die Vorrichtung entsprechend zur Aufnahme eines nächsten Projektionsbildes verfahren.

Es ergibt sich die Aufgabe, ein verbessertes Verfahren zum Betreiben eines Röntgengeräts für eine Erstellung eines dreidimensionalen Abbilds eines Körperteils bereitzustellen. Insbesondere soll das dreidimensionale Abbild dabei für die Berechnung eines Wertes einer fraktionellen Flussreserve geeignet sein.

Diese Aufgabe wird durch die Gegenstände der unabhängigen Patentansprüche gelöst. Vorteilhafte Ausführungsformen ergeben sich aus den abhängigen Ansprüchen, der Beschreibung und den Figuren.

Die Erfindung betrifft ein Verfahren zum Betreiben eines Röntgengeräts für ein Erstellen eines dreidimensionalen Abbilds eines Körperteils, insbesondere eines Blutgefäßes und/oder eines Blutgefäßteils und/oder eines Organs. Dieses Verfahren umfasst eine Reihe von Schritten, welche in der angegebenen Reihenfolge oder aber in einer anderen Reihenfolge vorgenommen werden können. Gegebenenfalls können einzelne Schritte auch wiederholt durchgeführt werden.

Ein Schritt ist hier das Bereitstellen einer ersten Röntgenaufnahme des (zu untersuchenden bzw. untersuchten) Körperteils an eine Recheneinheit des Röntgengerätes. Diese Röntgenaufnahme wurde dabei aus einem ersten Aufnahmewinkel aufgenommen. Die erste Röntgenaufnahme kann eine zuvor, beispielsweise auch für einen anderen Zweck, aufgenommene und in einem Datensystem hinterlegte Aufnahme sein. Die erste Röntgenaufnahme kann auch vor, insbesondere unmittelbar vor, dem Bereitstellen durch eine Aufnahmeeinheit des Röntgengeräts aufgenommen worden sein.

Ein weiterer Schritt ist ein automatisches Analysieren der bereitgestellten ersten Röntgenaufnahme durch die Recheneinheit. Das automatische Analysieren kann auch ein Auslesen eines ersten Aufnahmewinkels der ersten Röntgenaufnahme umfassen. Dieses automatische Analysieren kann auch durch ein manuelles Analysieren ergänzt werden. Dabei können die weiter unten angegebenen Merkmale des automatischen Analysierens alternativ oder ergänzend in dem manuellen Analysieren realisiert werden. Das automatische Analysieren kann auch ein teilautomatisches Analysieren umfassen, bei welchem beispielsweise durch eine Nutzerinteraktion wie ein Auswählen eines spezifischen Bildbereiches das automatische Analysieren nur mit Unterstützung durch eine Bedienperson möglich ist. Parallel zu dem automatischen Analysieren kann auch eine automatische Bildvorverarbeitung der ersten und/oder zweiten Röntgenaufnahme, beispielsweise für eine quantitative Analyse durch eine Bedienperson beziehungsweise einen Arzt, erfolgen.

Ein anderer Schritt ist das Bewerten der Eignung von zumindest einem weiteren Aufnahmewinkel, bevorzugt einer Vielzahl von weiteren Aufnahmewinkeln, für das Erstellen des dreidimensionalen Abbilds des Körperteils durch die Recheneinheit. Dies kann auch anhand des ersten Aufnahmewinkels erfolgen. Die Recheneinheit bewertet also, in welchem Ausmaß oder Grad ein oder mehrere weitere Aufnahmewinkel geeignet sind für das Erstellen des dreidimensionalen Abbilds in größtmöglicher Güte, beispielsweise in größtmöglicher Genauigkeit. Dabei berücksichtigt die Recheneinheit zumindest ein Ergebnis des automatischen Analysierens. Das Bewerten kann dabei anhand vorgegebener Kriterien erfolgen. Hier ist entscheidend, dass für das Erstellen des dreidimensionalen Abbilds (später, also nach dem Bewerten) eine zweite Röntgenaufnahme aus einem zweiten Aufnahmewinkel aufgenommen wird oder aufzunehmen ist.

Der zweite Aufnahmewinkel kann dann der Aufnahmewinkel, welcher bewertet wurde, oder einer der weiteren Aufnahmewinkel, welche bewertet wurden, sein.

Ein nächster Schritt ist ein Einstellen des zweiten Aufnahmewinkels an dem Röntgengerät. Dies kann automatisch durch die Recheneinheit erfolgen, wobei ein Ergebnis des Bewertens der Eignung des zweiten Aufnahmewinkels für das Erstellen des dreidimensionalen Abbilds berücksichtigt wird. Alternativ kann dies manuell durch eine Bedienperson erfolgen, wobei der Bedienperson ein Ergebnis des Bewertens der Eignung des zweiten Aufnahmewinkels zum Erstellen des dreidimensionalen Abbilds durch eine Anzeigeeinheit des Röntgengeräts angezeigt wird. Hierauf folgt ein manuell kontrolliertes Anfahren des eingestellten zweiten Aufnahmewinkels durch eine Aufnahmeeinheit des Röntgensystems als nächster Schritt sowie ein Aufnehmen der zweiten Röntgenaufnahme unter dem angefahrenen zweiten Aufnahmewinkel durch die Aufnahmeeinheit.

Ein weiterer Schritt kann hier auch ein automatisches Analysieren der zweiten Röntgenaufnahme durch die Recheneinheit sein. Dieses Analysieren kann hier dem Analysieren der ersten Röntgenaufnahme entsprechen.
Ebenfalls kann als weiterer Schritt ein Erstellen des dreidimensionalen Abbilds des Körperteils aus den beiden Röntgenaufnahmen durch die Recheneinheit sein.

Durch das Verfahren wird also einer Bedienperson, zum Beispiel einem Arzt bei dem Gewinnen von Daten für das Erstellen des dreidimensionalen Modells von Anfang an, also bereits bei dem Aufnehmen der erforderlichen Röntgenaufnahmen, auf welchen das Erstellen des dreidimensionalen Modells basiert, unterstützt. So wird auf einfache Weise eine verbesserte Qualität des resultierenden Modells erzielt und somit insbesondere eine genauere Berechnung eines fraktionellen Flussreservewertes möglich. Dabei können die aufgenommenen Röntgenaufnahmen oder Röntgenbilder auch parallel mehrfach verarbeitet werden, nämlich einerseits zur visuellen Betrachtung optimiert dargestellt werden und andererseits in einer für eine nachträgliche Bildbearbeitung oder Bildauswertung, beispielsweise eine densiometrische Auswertung, optimierten Version dargestellt oder abgespeichert oder der Recheneinheit zur Verfügung gestellt werden.

Es ergibt sich der Vorteil, dass die Bedienperson von dem System darin unterstützt wird, die optimale zweite Angulation, also den optimalen zweiten Aufnahmewinkel für das Erstellen des dreidimensionalen Modells einzustellen oder diesem nahezukommen. Damit kann gerade die zweite Röntgenaufnahme besser und schneller Einzelbilder liefern, welche zu einem genaueren dreidimensionalen Abbild des Körperteils führen. Das Verfahren ist auch weniger abhängig von einer Erfahrung oder einem Kenntnisstand der Bedienperson, was die Variabilität in der Qualität der Röntgenaufnahmen und damit des dreidimensionalen Modells sowie der auf dem Modell basierenden Berechnungen, beispielsweise der Berechnung des Wertes für die fraktionelle Flussreserve, verringert. Somit wird die Auswertung zuverlässiger. Durch das Bewerten der Eignung durch die Recheneinheit ist zugleich auch eine Qualitätskontrolle realisiert, da für den zweiten Aufnahmewinkel objektiv nachvollziehbare Eignungskriterien vorliegen.

In einer vorteilhaften Ausführungsform ist ein weiterer Verfahrensschritt ein Auswählen des "Erstellens eines dreidimensionalen Abbilds" als Bestimmungszweck für die erste Röntgenaufnahme aus einer Mehrzahl an möglichen Bestimmungszwecken für Aufnahmen des Röntgengeräts durch die Bedienperson an einer Bedieneinheit des Röntgengeräts. Das Auswählen entspricht hier also einem Auswählen einer vorgegebenen Option mit zumindest einer vorgegebenen Aufnahmeparameter-Einstellung für die Röntgenaufnahme aus einer Vielzahl von vorgegebenen Optionen mit je zumindest einer weiteren vorgegebenen Aufnahmeparameter-Einstellung für Röntgenaufnahmen an dem Röntgengerät.

Ein weiterer Schritt ist hier ein Einstellen des ersten Aufnahmewinkels für die erste Röntgenaufnahme. Das Einstellen kann sowohl durch die Bedienperson als auch durch die Recheneinheit erfolgen. Es wird auch automatisch zumindest ein weiterer Aufnahme- oder Akquisitionsparameter für die erste Röntgenaufnahme durch die Recheneinheit eingestellt. Der oder die Aufnahmeparameter kann dabei beispielsweise einen Aufnahmewinkel und/oder eine Intensität der Röntgenstrahlung bei der Aufnahme und/oder eine Position der Aufnahmeeinheit und/oder eine Betriebsspannung der Aufnahmeeinheit und/oder einen Betriebsstrom der Aufnahmeeinheit und/oder zumindest einen Parameter einer automatisch erfolgenden Bildvorverarbeitung umfassen.

Ein weiterer Schritt ist ein manuell kontrolliertes Anfahren des eingestellten ersten Aufnahmewinkels durch die Aufnahmeeinheit des Röntgensystems und ein Aufnehmen der ersten Röntgenaufnahme unter dem angefahrenen ersten Aufnahmewinkel durch die Aufnahmeeinheit. Bei der Aufnahmeeinheit kann es sich um eine bewegliche Aufnahmeeinheit wie zum Beispiel einen C-Bogen eines Röntgengeräts handeln.

Das hat den Vorteil, dass die für den Verwendungszweck bestmöglich geeigneten Aufnahmeparameter automatisch eingestellt werden und so auf besonders einfache Art und Weise die Eignung der ersten Röntgenaufnahme für das Erstellen des dreidimensionalen Abbilds verbessert wird. Somit wird erneut auf einfache Weise die Qualität des dreidimensionalen Abbilds verbessert.

Dabei kann insbesondere vorgesehen sein, dass das Auswählen auch ein Spezifizieren des abzubildenden Körper- oder Gefäßteils umfasst. Das Einstellen des ersten Aufnahmewinkels kann automatisch durch die Recheneinheit erfolgen oder manuell durch eine Bedienperson, wobei im letzteren Fall der Bedienperson basierend auf der ausgewählten Spezifikation des abzubildenden Körperteils eine Eignung des eingestellten ersten Aufnahmewinkels für das Erstellen des dreidimensionalen Abbilds durch die Anzeigeeinheit des Röntgengerätes wird. Dies kann entsprechend der Merkmale erfolgen, welche für das Einstellen des zweiten Aufnahmewinkels weiter oben beschrieben sind. Insbesondere kann auch zumindest ein weiterer Aufnahmeparameter automatisch eingestellt werden. Bei dem automatischen Einstellen des ersten Aufnahmewinkels kann hier in der Recheneinheit ein Modell des abzubildenden Körperteils und/oder dessen Umgebung im Körper hinterlegt werden. Somit können bestimmten Auswahlen, beispielsweise die aufgrund einer üblicherweise vorliegenden Geometrie von Blutgefäßen vorteilhaften Aufnahmewinkel bevorzugt eingestellt werden können. Es können beispielsweise Angulationen, bei welchen weniger Überlagerungen des Körperteils, welche zu unerwünschten Schatten in der Aufnahme führen, auftreten, bevorzugt werden.

Das hat den Vorteil, dass das Verfahren weiter vereinfacht wird, sodass der Arzt nur noch eine vorgegebene Angulation beziehungsweise einen vorgegebenen ersten Aufnahmewinkel anfahren muss beziehungsweise bei dem Einstellen des ersten Aufnahmewinkels nur noch einen Hinweis oder eine Empfehlung der Recheneinheit beachten muss. Erneut wird das Verfahren hier vereinfacht und durch eine geschickte Wahl des ersten Aufnahmewinkels die Qualität und Eignung der ersten Röntgenaufnahme für das Erstellen des dreidimensionalen Abbilds verbessert.

In einer weiteren vorteilhaften Ausführungsform ist vorgesehen, dass das Aufnehmen bei der ersten und bei der zweiten Röntgenaufnahme ein automatisches Variieren des Aufnahmewinkels während des Aufnehmens um wenige Grad um den eingestellten Aufnahmewinkel herum umfasst. Insbesondere kann der Aufnahmewinkel hier um weniger als 10°, bevorzugt um weniger als 5° und besonders bevorzugt um weniger als 2° variiert werden. Das hat den Vorteil, dass ein sich überlappendes Gefäßsystem oder Körperteilsystem unter verschiedenen, geringfügig unterschiedlichen Winkeln betrachtet werden kann und so eine verbesserte Chance besteht, ein überlagerungsfreies Bild oder ein überlagerungsarmes Bild, in welchem der gewünschte Körperteil oder das gewünschte Gefäßteil nur von wenig oder keinem anderen Körperteil oder Gefäßteil überlappt wird, besteht. Erfahrungsgemäß reicht hier eine Änderung des Aufnahmewinkels um wenige Grad, um derlei Überlagerungen zu vermeiden, welche beispielsweise eine automatische Kantenerkennung von mit Kontrastmitteln gefüllten Gefäßen erschweren, wenn beispielsweise kleine Gefäße das eigentlich zu erkennende Gefäß kreuzen. Da eine derartige geringfügige Variation des Aufnahmewinkels jedoch an sich das Erstellen eines dreidimensionalen Bildes aus zwei zweidimensionalen Einzelbildern kaum beeinflusst, überwiegt hier im dreidimensionalen Abbild insgesamt der Gewinn an Qualität. Auch kann so vermieden werden, dass in der späteren Bearbeitung für das Erstellen des dreidimensionalen Modells ein anderes Einzelbild, beispielsweise einer weniger günstigen Herzphase zuzuordnendes Einzelbild, ausgewählt werden muss. Auch kann beispielsweise eine durch eine Bedienperson eine geschätzte Korrektur unterbleiben. Auch so wird wieder die Genauigkeit, Einfachheit und Nachvollziehbarkeit des Verfahrens erhöht.

In einer weiteren vorteilhaften Ausführungsform ist vorgesehen, dass das automatische Analysieren ein automatisches Detektieren zumindest eines Bereichs des Körperteils mit vorgegebenen Eigenschaften, insbesondere ein Bereich eines Blutgefäßes mit einer Stenose, und/oder einer Position des Körperteils, insbesondere einer Position des Körperteils auf der Röntgenaufnahme in Bezug auf einen Aufnahmeausschnitt, insbesondere einen Abstand zu einem Bildmittelpunkt oder Isocenter, und/oder einer Orientierung des Körperteils und/oder zumindest einer Abmessung des Körperteils, insbesondere einer für eine fraktionelle Flussreserve ausreichende geometrischen Größe, und/oder eines Verlaufs des Körperteils und/oder einer Rotationsachse, um welche die zweite Röntgenaufnahme gegenüber der ersten Röntgenaufnahme für ein Erstellen des dreidimensionalen Abbilds des Körperteils optimalerweise verkippt wird, umfasst. Die Rotationsachse verläuft dabei insbesondere parallel zur Bildebene der ersten Röntgenaufnahme. Bevorzugt verläuft die Rotationsachse in der Bildebene der ersten Röntgenaufnahme. Optimalerweise werden hier insbesondere die Rotationsachse und der Aufnahmewinkel der zweiten Röntgenaufnahme so gewählt, dass die Bildebene der zweiten Röntgenaufnahme senkrecht zur Bildebene der ersten Röntgenaufnahme und senkrecht zu einem Verlauf des Körperteils steht.

Das automatische Analysieren kann auch ein Auswählen eines Einzelbildes aus der Aufnahme umfassen. Beispielsweise kann ein Einzelbild mit einer für den Bestimmungszweck, vorliegend beispielsweise das Erzeugen eines dreidimensionalen Abbildes, geeigneten Herzphase und/oder eines geeigneten Füllungsgrades des Körperteils mit Blut umfassen. Das Detektieren des zumindest einen Bereichs des Körperteils mit vorgegebenen Eigenschaften kann beispielsweise über einen Kantendetektions- und/oder Segmentierungsalgorithmus in der Recheneinheit erfolgen. Alternativ kann das Detektieren ganz oder teilweise auch durch die Bedienperson erfolgen oder unterstützt werden. Beispielsweise kann durch eine Bedienhandlung wie ein Klicken auf einen Bildbereich ein Bereich eines Körperteils mit den vorgegebenen Eigenschaften in die Recheneinheit eingegeben werden oder so ein automatisches Analysieren weiterer Bildbereiche unterstützt werden.

Auch dies bietet den Vorteil, dass so automatisch oder teilautomatisch die Auswahl des zweiten Aufnahmewinkels für die zweite Röntgenaufnahme zum Erstellen des dreidimensionalen Abbilds verbessert wird. Es wird somit die Genauigkeit und Zuverlässigkeit es dreidimensionalen Abbilds und insbesondere der Untersuchung der funktionellen Flussreserve erhöht.

In einer weiteren Ausführungsform ist vorgesehen, dass das vorgegebene Kriterium für das Bewerten der Eignung des Aufnahmewinkels eines oder mehrere der folgenden Kriterien umfasst: eine Winkeldifferenz zwischen dem ersten Aufnahmewinkel und dem zumindest einen weiteren Aufnahmewinkel, eine Kollisionswahrscheinlichkeit des Röntgengeräts, insbesondere der Aufnahmeeinheit, mit weiteren Geräten wie beispielsweise einem Patiententisch des Röntgengeräts und/oder der Bedienperson und/oder dem Patienten, eine gemäß einem in der Recheneinheit hinterlegten Patientenmodell oder Körperteilmodell auftretende Dosisbelastung für den Patienten, eine Anfahrbarkeit des Aufnahmewinkels, insbesondere aus einer aktuellen Position der Aufnahmeeinheit durch die Aufnahmeeinheit, eine gemäß einem in der Recheneinheit hinterlegten Modell des Patienten oder des Körperteils in dem Patienten und/oder dem Körperteil zu durch die Röntgenstrahlung zurückgelegte Weglänge oder eine gemäß einem in der Recheneinheit hinterlegten Modell des Körperteils und/oder weiterer Körperteile auftretende Anzahl oder Ausmaß von Überlagerungen weiterer Körperteile mit dem Körperteil in der Röntgenaufnahme. Die Anfahrbarkeit kann dabei beispielsweise anhand einer für das Anfahren des Aufnahmewinkels Anzahl der zu bewegenden Komponenten oder einer für das Anfahren des Aufnahmewinkels erforderlichen Zeit bewertet werden.

Alternativ kann das Bewerten der Eignung auch geschätzt werden. Dabei kann eines oder mehrerer der genannten Kriterien nach einem Spezifizieren des dreidimensional abzubildenden Körperteils auch ohne ein Analysieren oder nur nach einem geringstmöglich ausgeprägten Analysieren der ersten Röntgenaufnahme abgeschätzt werden. Das Abschätzen kann basierend auf einem in der Recheneinheit hinterlegten Modell erfolgen. Insbesondere können hier auch Methoden des Maschinenlernens zum Einsatz kommen.

Die genannten Kriterien haben den Vorteil, dass sie sich besonders stark auf eine Qualität des dreidimensionalen Abbilds auswirken beziehungsweise andere erstrebenswerte Ziele realisieren wie eine bestmöglich zu erhaltende Gesundheit des Patienten und/oder eine Geschwindigkeit, mit der der Aufnahmewinkel durch die Aufnahmeeinheit erreicht werden kann.

Bei der vorliegenden Erfindung ist vorgesehen, dass das Bewerten für das vorgegebene Kriterium zumindest eine Mindestanforderung berücksichtigt und mittels der Recheneinheit bei einem Nichterfüllen der Mindestanforderung durch den bewerteten Aufnahmewinkel das Einstellen von dem bewerteten Aufnahmewinkel als zweiten Aufnahmewinkel verhindert wird und/oder die Bedienperson bei dem Einstellen von dem bewerteten Aufnahmewinkel als zweiten Aufnahmewinkel gewarnt wird.

Dies hat den Vorteil, dass die Effizienz des Verfahrens erhöht wird. Fehler, welche die Bedienperson vornehmen könnte, werden frühzeitig erkannt und somit ein schnelles Erstellen des dreidimensionalen Abbilds erzielt sowie unnötige zusätzliche Aufnahmen vermieden.

Dabei kann insbesondere vorgesehen sein, dass die Mindestanforderung einen vorgegebenen Winkelbetrag umfasst, um welchen sich der erste und der zweite Aufnahmewinkel unterscheiden muss. Insbesondere kann der Differenzwinkel zwischen den beiden Aufnahmen, welcher sich aus dem ersten und zweiten Aufnahmewinkel ergibt, und um welchen sich die beiden Aufnahmewinkel mindestens unterscheiden müssen, 20° oder 30° oder 40° betragen. Das hat den Vorteil, dass das dreidimensionale Abbild eine vorgegebene Mindestqualität nicht unterschreitet.

In einer weiteren vorteilhaften Ausführungsform ist vorgesehen, dass das Röntgengerät einen Patiententisch für den Patienten mit dem dreidimensional abzubildenden Körperteil mit einer elektrischen Verstellfunktion umfasst. Dabei wird die Verstellfunktion während des Aufnehmens der ersten oder zweiten Röntgenaufnahme deaktiviert, sodass eine Bedienperson den Patiententisch nicht mehr verstellen kann. Insbesondere wird in dem Zeitraum zwischen den beiden Röntgenaufnahmen entweder die Verstellfunktion deaktiviert oder ein Verstellen des Patiententisches durch eine Detektiereinheit detektiert und bei dem Einstellen des zweiten Aufnahmewinkels durch die Recheneinheit berücksichtigt. Insbesondere kann das Deaktivieren der elektrischen Verstellfunktion an eine Wahl des "Erstellens eines dreidimensionalen Abbilds" aus einer Mehrzahl an möglichen Bestimmungszwecken, wie es oben beschrieben ist, gekoppelt sein. Das hat den Vorteil, dass die räumliche Relation der beiden Röntgenaufnahmen zueinander, welche entscheidend für die Qualität des dreidimensionalen Abbilds ist, definiert und somit bei dem Erstellen des dreidimensionalen Abbilds bekannt ist. Somit kann die Genauigkeit des dreidimensionalen Abbilds auf einfache Weise verbessert werden.

In einer weiteren vorteilhaften Ausführungsform ist vorgesehen, dass durch die Recheneinheit unter Berücksichtigung von Ergebnissen des automatischen Analysierens und/oder des Bewertens der Eignung der jeweiligen Röntgenaufnahme oder Röntgenaufnahmen für das Erstellen des dreidimensionalen Abbilds ein automatisches Vorauswählen von korrelierten Einzelbildern bzw. Einzelbildpaaren der beiden Röntgenaufnahmen erfolgt. Korreliert sind hier beispielsweise Einzelbilder der unterschiedlichen Röntgenaufnahmen, wenn sie der gleichen Herzphase und/oder dem gleichen Füllungsgrad des dort aufgenommenen Körperteils entsprechen. Auch dies ergibt den Vorteil, dass die bereits im System verfügbare und hinterlegte Information genutzt wird, um der Bedienperson die bestmögliche Unterstützung zukommen zu lassen und unabhängig oder möglichst unabhängig von deren Kenntnisstand auf einfache Weise ein hochqualitatives dreidimensionales Abbild des Körperteils zu erlangen.

Überdies kann auch vorgesehen sein, nach dem Erstellen des dreidimensionalen Abbilds des Körperteils aus den beiden Röntgenaufnahmen retrospektiv die Aufnahmewinkel für den Körperteil beziehungsweise das Gefäßteil oder Gefäßsegment zu analysieren. Dies ist möglich, da ja aus dem dreidimensionalen Modell ist Lage des Körperteils in Bezug auf eine Position der Aufnahmeeinheit, beispielsweise eines C-Bogens, bekannt ist. Sind nun beispielsweise aufgrund von Krümmungen des Körperteils einzelne Teile unter einem ungünstigen Winkel aufgenommen, können diese Bereiche, in welchen somit bekanntermaßen das dreidimensionale Abbild eine verringerte Genauigkeit aufweist, zum Beispiel farblich, markiert werden und damit ein Hinweis auf eine in diesen Bereich möglicherweise auch verringerte Genauigkeit der fraktionellen Flussreserveauswertung angezeigt werden. Auch dies dient der vereinfachten Realisierung einer Qualitätskontrolle.

In einer weiteren vorteilhaften Ausführungsform ist vorgesehen, dass nach dem Aufnehmen der zweiten Röntgenaufnahme, insbesondere nach dem Erstellen des dreidimensionalen Abbilds des Körperteils, ein Analysieren der zweiten Röntgenaufnahme, insbesondere des dreidimensionalen Abbilds, erfolgt. Das Analysieren kann hier auch ein automatisches Analysieren sein. Des Weiteren erfolgt hier auch ein Bewerten der Eignung von zumindest einem weiteren Aufnahmewinkel für das Erstellen eines verbesserten dreidimensionalen Abbilds des Körperteils durch die Recheneinheit. Das Bewerten erfolgt hier unter Berücksichtigung eines Ergebnisses des Analysierens. Für das Erstellen des verbesserten dreidimensionalen Abbilds wird eine dritte Röntgenaufnahme aus einem dritten Aufnahmewinkel aufgenommen, sowie die bereits für die zweite Röntgenaufnahme bekannten Schritte des Einstellens, des manuell kontrollierten Anfahrens sowie des Aufnehmens für die dritte Röntgenaufnahme durchgeführt wird. Gegebenenfalls können hier auch weitere der für die erste und/oder zweite Röntgenaufnahme bekannten Schritte für die dritte Röntgenaufnahme durchgeführt werden. Insbesondere kann für die dritte Röntgenaufnahme ein Aufnahmewinkel eingestellt werden, welcher mit der ersten oder mit der zweiten Röntgenaufnahme kombiniert ein Erstellen eines optimalen, also bestmöglichen dreidimensionalen Abbilds des Körperteils ermöglicht. Eine optimale Kombination von Aufnahmewinkeln kann bestimmt werden, da die Recheneinheit die bisherigen Aufnahmewinkel und aus dem Modell auch den Verlauf des Körperteils kennt.

Das hat den Vorteil, dass für die erste retrospektiv bekannte tatsächliche Position und Orientierung des abzubildenden Körperteils ein optimaler Aufnahmewinkel eingestellt werden kann, und auf einfache Weise ein bestmögliches dreidimensionales Abbild erstellt werden kann.

Die Erfindung betrifft auch ein Röntgengerät zum Aufnehmen von zumindest zwei Röntgenbildern für ein Erstellen eines dreidimensionalen Abbilds eines Körperteils. Das Röntgengerät weist dabei eine Aufnahmeeinheit zum Aufnehmen von Röntgenaufnahmen aus zumindest einem, also einem oder mehreren, an dem Röntgengerät einstellbaren und durch die Aufnahmeeinheit unter manueller Kontrolle anfahrbaren Aufnahmewinkel(n) auf. Des Weiteren hat das Röntgengerät eine Recheneinheit zum automatischen Analysieren von zumindest einer an die Recheneinheit bereitgestellten ersten Röntgenaufnahme, welche aus einem ersten Aufnahmewinkel aufgenommen ist. Die Recheneinheit ist auch ausgelegt zum Bewerten einer Eignung von zumindest einem weiteren Aufnahmewinkel beziehungsweise einer Vielzahl von weiteren Aufnahmewinkeln für das Erstellen des dreidimensionalen Abbilds des Körperteils unter Berücksichtigung eines Ergebnisses des automatischen Analysierens. Dabei ist für das Erstellen des dreidimensionalen Abbilds zumindest eine zweite Röntgenaufnahme aus zumindest einem zweiten Aufnahmewinkel durch die Aufnahmeeinheit aufnehmbar.

Vorteile und vorteilhafte Ausführungsformen des Röntgengeräts entsprechen hier den Vorteilen und vorteilhaften Ausführungsformen des Verfahrens zum Betreiben eines Röntgengeräts für ein Erstellen eines dreidimensionalen Abbilds eines Körperteils.

Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder abweichen.

Ausführungsbeispiele der Erfindung werden nachfolgend anhand schematischer Zeichnungen näher erläutert. Dabei zeigen:
- FIG 1: ein Flussdiagramm eines beispielhaften Verfahrens zum Betreiben eines Röntgengeräts für ein Erstellen eines dreidimensionalen Abbildes eines Körperteils;
- FIG 2: eine beispielhafte erste Röntgenaufnahme aus einem ersten Aufnahmewinkel zum Visualisieren des Analysierens;
- FIG 3: eine schematische dreidimensionale Darstellung des ersten Röntgenbildes aus FIG 2 zum Visualisieren des Bewertens der Eignung von einem weiteren Aufnahmewinkel für das Erstellen des dreidimensionalen Abbilds; und
- FIG 4: eine beispielhafte Ausführungsform eines Röntgengeräts.

In den Figuren werden gleiche oder funktionsgleiche Elemente mit den gleichen Bezugszeichen versehen.

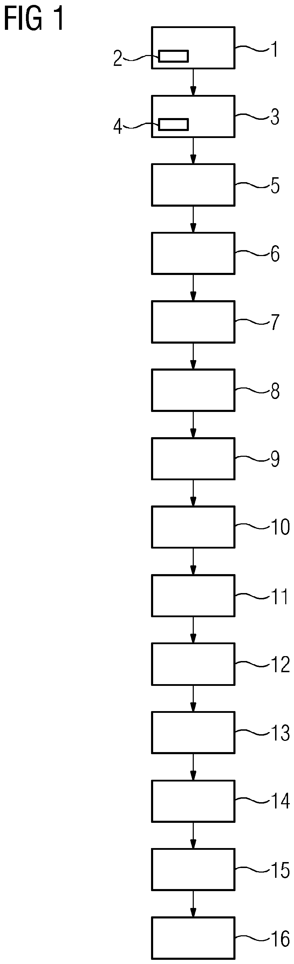

FIG 1 zeigt eine schematische Darstellung eines Flussdiagramms eines beispielhaften Verfahrens zum Betreiben eines Röntgengeräts für ein Erstellen eines dreidimensionalen Abbilds eines Körperteils. Vorliegend erfolgt in einem ersten Schritt ein Auswählen eines spezifischen Aufnahmeprogramms des Röntgengeräts, nämlich das "Erstellen eines dreidimensionalen Abbilds" durch eine Bedienperson, beispielsweise einen Arzt. Im gezeigten Beispiel umfasst das Auswählen auch ein Spezifizieren 2 des abzubildenden Körperteils. So wählt der Arzt im gezeigten Beispiel aus einer Mehrzahl an möglichen Bestimmungszwecken für Aufnahmen des Röntgengeräts als Bestimmungszweck die Option "Erstellen eines dreidimensionalen Abbilds einer rechten Koronararterie", kurz beispielsweise "3D-Modell RCA".

Im gezeigten Beispiel ist ein automatisches Einstellen 3 der für das Erstellen eines dreidimensionalen Abbilds besten Aufnahme- oder Akquisitionsparameter die Folge. Beispielsweise kann eine Strahlungsintensität automatisch eingestellt werden. Vorliegend umfasst das automatische Einstellen 3 des Aufnahmeparameters auch ein automatisches Einstellen 4 des ersten Aufnahmewinkels. Entsprechend dem gewählten Körperteil wird hier automatisch eine vorliegend für die Aufnahme der rechten Koronararterie geeignete Angulation, also ein geeigneter Aufnahmewinkel, eingestellt.

Der Arzt führt nunmehr nur noch das manuell kontrollierte Anfahren 5 des eingestellten ersten Aufnahmewinkels durch, worauf das Aufnehmen 6 der ersten Röntgenaufnahme erfolgt.

Im gezeigten Beispiel erfolgt nunmehr nach einem Bereitstellen 7 der ersten Röntgenaufnahme an eine Recheneinheit ein Analysieren 8 der ersten Röntgenaufnahme durch den Arzt. Das Analysieren 8 umfasst vorliegend die Wahl eines Einzelbildes aus der ersten Röntgenaufnahme mit einer geeigneten Herzphase. Des Weiteren umfasst es ein Definieren der Stenose durch den Arzt, beispielsweise durch ein Anklicken oder Markieren des betrachteten Gefäßsegmentes auf einer Anzeigeeinheit des Röntgengeräts, beispielsweise einem Bildschirm. Des Weiteren umfasst das Analysieren 8 des Arztes hier auch ein Kontrollieren, ob das zu betrachtende Gefäßsegment, hier die rechte Koronararterie, überlagerungsfrei von anderen Blutgefäßen oder Organen detektiert wird. Ist dies nicht der Fall, kann durch den Arzt eine Korrektur der Aufnahmeparameter und/oder des ersten Aufnahmewinkels erfolgen sowie gegebenenfalls auch weitere Schritte.

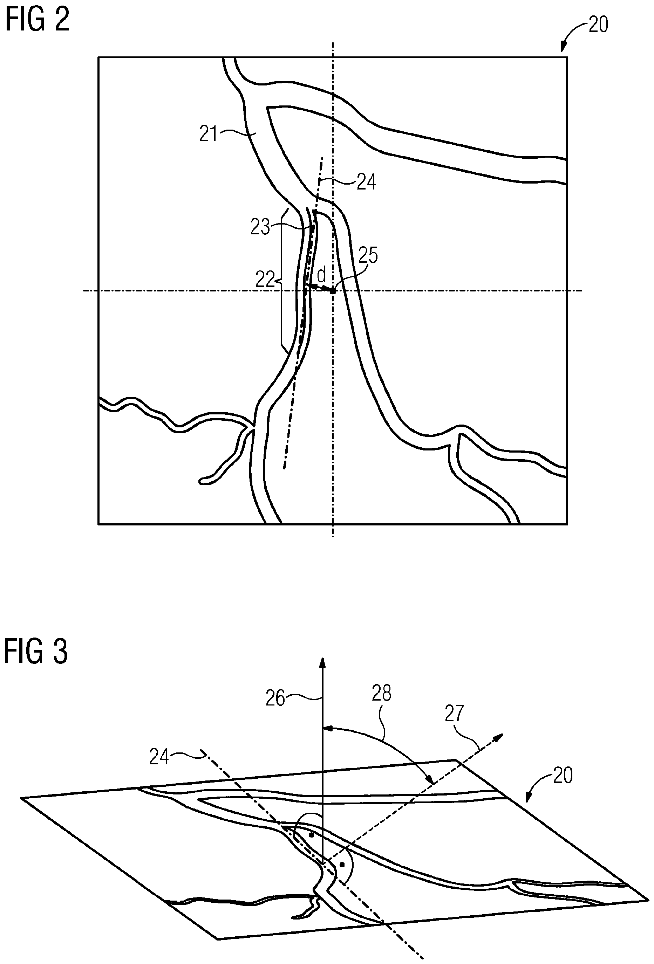

In einem darauffolgenden Schritt erfolgt nun ein automatisches Analysieren 9 der ersten Röntgenaufnahme durch die Recheneinheit. Dies umfasst beispielsweise eine Kantenerkennung sowie die Erkennung der Erstreckung des zu betrachtenden Gefäßes beziehungsweise des Gefäßsegments mit der Stenose. Aus diesen kann eine Mittellinie 23 (FIG 2), welche sich entlang des Verlaufs des von der Stenose betroffenen Gefäßsegments erstreckt, berechnet werden. An diese Mittellinie 23 kann eine Gerade als Rotationsachse 24 (FIG 2)für den Aufnahmewinkel der zweiten Röntgenaufnahme angenähert werden.

Ist die Mittellinie 23 stark gekrümmt, kann hier ein Hinweis an die Bedienperson erfolgen, dass der zweite Aufnahmewinkel sich nur bedingt optimal einstellen lässt. Es kann in diesem Fall auch die Rotationsachse 24 als eine Tangente an die Mittellinie 23 im Bereich der Stenose gewählt werden. Ist die Stenose sehr lang, und Teil der Krümmung beziehungsweise ungünstig an einer Bifurkation des Körperteil oder Blutgefäßes gelegen, kann von der Recheneinheit eine zusätzliche dritte Angulation, also ein weiterer Aufnahmewinkel für eine dritte Röntgenaufnahme vorgeschlagen werden.

Das automatische Analysieren erfolgt vorliegend unter der Annahme, dass die Stenose im Wesentlichen parallel zur Bildebene der ersten Röntgenaufnahme verläuft, also der erste Aufnahmewinkel im Wesentlichen senkrecht zu der Stenose beziehungsweise deren Verlauf gemacht wurde. Im nächsten Schritt des Bewertens 10 kann ausgehend hiervon der optimale zweite Aufnahmewinkel für die zweite Röntgenaufnahme als senkrecht zu der Mittellinie 23 und zu dem ersten Aufnahmewinkel angenommen werden. Parallel wird vorliegend von der Recheneinheit jedoch überprüft, ob der optimale zweite Aufnahmewinkel überhaupt anfahrbar und somit eine zweite Röntgenaufnahme aus dem zweiten Aufnahmewinkel überhaupt möglich ist. Bei dem Bewerten können auch weitere Kriterien berücksichtigt werden, vorliegend beispielsweise ein Überdeckungsgrad des spezifizierten Körperteils durch andere Teile, welcher gemäß einem in der Recheneinheit hinterlegten Modell auftritt.

Entsprechend kann so die Eignung einer Vielzahl von vorgegebenen weiteren Aufnahmewinkeln überprüft werden. Nach einem Ausschluss beispielsweise der nicht anfahrbaren oder sonstige Mindestanforderungen nicht erfüllenden Aufnahmewinkel kann der weitere Aufnahmewinkel mit dem besten Bewertungsergebnis automatisch von der Recheneinheit eingestellt werden. Beispielsweise können Aufnahmewinkel mit einem einen vorgegebenen Mindestdifferenzwinkel, zum Beispiel 40 Grad, unterschreitenden Differenzwinkel zwischen den beiden Aufnahmewinkeln von der Menge an möglichen zweiten Aufnahmewinkeln ausgenommenen werden.

Im gezeigten Beispiel wird dann automatisch von der Recheneinheit aus einer Menge an weiteren Aufnahmewinkeln der zweite Aufnahmewinkel für die zweite Röntgenaufnahme ausgewählt. Dieser wird dann hier einer Bedienperson angezeigt, damit diese in als nächsten Schritt Einstellen 11 kann. Nach dem Einstellen 11 erfolgt dann das Anfahren 12 des zweiten Aufnahmewinkels sowie das Aufnehmen 13 der zweiten Röntgenaufnahme unter dem angefahrenen zweiten Aufnahmewinkel.

In einem weiteren Schritt erfolgt nun ein automatisches Analysieren 14 der zweiten Röntgenaufnahme. Beispielsweise kann hier aus der zweiten Röntgenaufnahme das geeignete Einzelbild mit der zur Herzphase des aus der ersten Röntgenaufnahme gewählten Einzelbilds ausgewählt werden. Dies ist problemlos automatisch möglich, nachdem die Herzphase bereits aus dem Analysieren 8 der ersten Röntgenaufnahme des Arztes für das Einzelbild der ersten Röntgenaufnahme bekannt ist. Beispielsweise kann auch automatisch aus den Einzelbildern mit einer passenden Herzphase das Einzelbild mit dem höchsten Füllungsgrad des Körperteils, hier der rechten Koronararterie, an Kontrastmittel angezeigt werden. Auch kann ein automatisches Definieren der Stenose beziehungsweise des zu betrachtenden Körperteils oder Gefäßsegmentes durch die aus der ersten Röntgenaufnahme bekannten Epipolarlinien erfolgen. Optional kann der Arzt in einem zusätzlichen, hier nicht gezeigten, Schritt auch nochmals kontrollieren, ob das Körperteil oder Gefäßsegment überlagerungsfrei von anderen Gefäßen detektiert wird sowie gegebenenfalls eine Korrektur wie eine Aufnahmewinkeländerung vornehmen. Auch eine Registrierung der beiden Röntgenaufnahmen zueinander kommt hier als Korrektur infrage.

Es folgt als nächster Schritt ein Erstellen 15 des dreidimensionalen Abbilds des Körperteils, hier der rechten Koronararterie. Ist das dreidimensionale Abbild oder Modell einmal bekannt und damit die Lage des Körperteils im Raum gegeben, so kann nachträglich auch ein Vergleich der tatsächlichen Aufnahmewinkel mit optimalen Aufnahmewinkeln erfolgen und so die Qualität der Röntgenaufnahmen und damit der dreidimensionalen Darstellung ermittelt werden. Dies kann beispielsweise automatisch über einen Abgleich der jeweiligen Mittellinien in den Röntgenaufnahmen beziehungsweise den Einzelbildern und der Mittellinie im dreidimensionalen Modell erfolgen. Als Ergebnis kann ein Ausgeben 16 einer insbesondere ortsaufgelösten Güte des dreidimensionalen Abbilds abhängig von den Aufnahmewinkeln berechnet und ausgegeben werden. Gegebenenfalls kann durch die Recheneinheit auch das Aufnehmen einer weiteren Röntgenaufnahme mit einem Vorschlag für einen optimalen Aufnahmewinkel vorgeschlagen werden.

FIG 2 zeigt eine beispielhafte erste Röntgenaufnahme zur Illustration des automatischen Analysierens einer bereitgestellten Röntgenaufnahme durch die Recheneinheit. Vorliegend ist auf der in diesem Beispiel quadratischen ersten Röntgenaufnahme 20 ein Körperteil 21, beispielsweise ein Blutgefäß, dargestellt. In einem Bereich des Körperteils 21 ist nun vorliegend, sei es durch eine Bedienperson oder durch eine Recheneinheit, in einem Bereich 22 eine Stenose detektiert. Im Rahmen des automatischen Analysierens 9 der Röntgenaufnahme 20 wird nun in dem Bereich automatisch eine Mittellinie 23 berechnet, die dem Verlauf des von der Stenose betroffenen Körperteils 21 in den Bereich 22 folgt. Über einfache Rechenoperationen kann nun durch die Mittellinie 23 (Centerline) eine Gerade gelegt werden. Diese Gerade dient in diesem Beispiel zugleich als Rotationsachse 24, zu der eine Normale der Bildebene einer zweiten Röntgenaufnahme, die dem Erstellen eines dreidimensionalen Abbilds des Körperteils 21 dient, idealerweise senkrecht stehen sollte. Für die Rotationsachse 24 kann auch noch ein Abstand d von dem Mittelpunkt 25 (Isocenter) der ersten Röntgenaufnahme bestimmt werden.

In FIG 3 ist nun eine perspektivische Ansicht der Röntgenaufnahme aus FIG 2 zu sehen. Eingezeichnet ist hier nun zusätzlich der Normalenvektor 26 der Bildebene der Röntgenaufnahme 20. Der Normalenvektor 27 einer Bildebene für die zweite Röntgenaufnahme sollte nun gegenüber der Normalen 26 um einen vorgegebenen Mindestdifferenzwinkel verkippt sein. Dabei schneiden sich die beiden Bildebenen vorliegend in Rotationsachse 24. Ein Differenzwinkel 28 zwischen den beiden Normalen 26, 27 entspricht damit also (mit einer kleinen Korrektur, s.u.) dem Unterschied zwischen dem ersten und dem zweiten Aufnahmewinkel. Idealerweise beträgt der Differenzwinkel 28 im Allgemeinen ca. 90°, also 90° +/- 10°, insbesondere +/-5° .

Differenzwinkel 28 zwischen der ersten und der zweiten Normalen 26, 27 bezüglich der Rotation um die Rotationsachse 24 ändert sich bei einem Rotieren der Aufnahmeeinheit des Röntgengerätes relativ zu dem Bereich 22 des Körperteils anders, als der Aufnahmewinkel selber. Dies ist darauf zurückzuführen, dass der Bereich 22 im Allgemeinen nicht im Bildmittelpunkt 25 liegt. Dies macht gegebenenfalls eine Kompensation erforderlich. Beispielsweise kann in Abhängigkeit von dem Abstand d der Rotationsachse 24 zu dem Bildmittelpunkt 25 zu dem Differenzwinkel 28 ein Ausgleichbetrag hinzugezählt oder abgezogen werden.

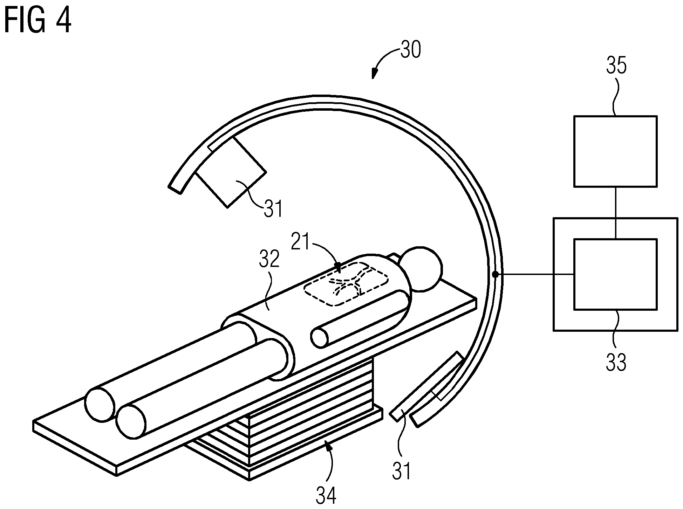

FIG 4 zeigt eine schematische Darstellung einer beispielhaften Ausführungsform der Erfindung. Das Röntgengerät 30 zum Aufnehmen von zumindest zwei Röntgenaufnahmen 20 (FIG 2) für das Erstellen eines dreidimensionalen Abbilds eines Körperteils 21 umfasst hier eine Aufnahmeeinheit 31 zum Aufnehmen von Röntgenaufnahmen 20 aus an dem Röntgengerät 30 einstellbaren und durch die Aufnahmeeinheit 31 unter manueller Kontrolle anfahrbaren Aufnahmewinkeln. Das Röntgengerät 30 umfasst auch eine Recheneinheit 33 zum automatischen Analysieren 9 von zumindest einer an die Recheneinheit 33 bereitgestellten ersten Röntgenaufnahme 20, welche aus einem ersten Aufnahmewinkel aufgenommen ist, und zum Bewerten 10 einer Eignung von zumindest einem weiteren Aufnahmewinkel für das Erstellen 15 des dreidimensionalen Abbilds des Körperteils 21 unter Berücksichtigung eines Ergebnisses des automatischen Analysierens 9. Ebenfalls umfasst das Röntgengerät 30 hier einen Patiententisch 34 für einen Patienten 32 mit einer elektrischen Verstellfunktion.

## Patentansprüche

1. Verfahren zum Betreiben eines Röntgengerätes (30) für ein Erstellen (15) eines dreidimensionalen Abbilds eines Körperteils (21), mit den Schritten:
- Bereitstellen (7) einer ersten Röntgenaufnahme (20) des Körperteils (21), welche aus einem ersten Aufnahmewinkel aufgenommen wurde, an eine Recheneinheit (33) des Röntgengerätes (30);
- automatisches Analysieren (9) der bereitgestellten ersten Röntgenaufnahme (20) durch die Recheneinheit (33);
- Bewerten (10) der Eignung von zumindest einem weiteren Aufnahmewinkel für das Erstellen (15) des dreidimensionalen Abbilds des Körperteils (21), durch die Recheneinheit (33) unter Berücksichtigung eines Ergebnisses des automatischen Analysierens (9), wobei für das Erstellen (15) des dreidimensionalen Abbildes eine zweite Röntgenaufnahme aus einem zweiten Aufnahmewinkel aufgenommen wird;
- Einstellen (11) des zweiten Aufnahmewinkels an dem Röntgengerät (30), entweder automatisch durch die Recheneinheit (33), wobei ein Ergebnis des Bewertens (10) berücksichtigt wird, oder manuell durch eine Bedienperson, wobei der Bedienperson ein Ergebnis des Bewertens (10) durch eine Anzeigeeinheit (35) des Röntgengerätes (30) angezeigt wird;
- manuell kontrolliertes Anfahren (12) des eingestellten zweiten Aufnahmewinkels durch eine Aufnahmeeinheit (31) des Röntgengeräts (30);
- Aufnehmen (13) der zweiten Röntgenaufnahme (20) unter dem angefahrenen zweiten Aufnahmewinkel durch die Aufnahmeeinheit (31),
**dadurch gekennzeichnet, dass**
- das Bewerten (7) der Eignung für ein vorgegebenes Kriterium zumindest eine Mindestanforderung berücksichtigt und mittels der Recheneinheit (33) bei einem Nichterfüllen der Mindestanforderung durch den bewerteten Aufnahmewinkel das Einstellen (11) von dem bewerteten Aufnahmewinkel als zweiten Aufnahmewinkel verhindert wird und/oder die Bedienperson bei einem Einstellen (11) von dem bewerteten Aufnahmewinkel als zweitem Aufnahmewinkel gewarnt wird.

2. Verfahren nach Anspruch 1,
**gekennzeichnet durch** ein
- Auswählen (1) des "Erstellens eines dreidimensionalen Abbilds" aus einer Mehrzahl an möglichen Bestimmungszwecken für Aufnahmen des Röntgengerätes (30) als Bestimmungszweck für die erste Röntgenaufnahme (20) durch die Bedienperson;
- automatisches Einstellen (3) zumindest eines Aufnahmeparameter für die erste Röntgenaufnahme (20);
- Einstellen (4) des ersten Aufnahmewinkels;
- manuell kontrolliertes Anfahren (5) des eingestellten ersten Aufnahmewinkels durch die Aufnahmeeinheit (31) des Röntgengeräts (30);
- Aufnehmen (6) der ersten Röntgenaufnahme (20) unter dem angefahrenen ersten Aufnahmewinkel durch die Aufnahmeeinheit (31).

3. Verfahren nach Anspruch 2,
**dadurch gekennzeichnet, dass**
- das Auswählen (1) auch ein Spezifizieren (2) des abzubildenden Körperteils (32) umfasst und
- das Einstellen (4) des ersten Aufnahmewinkels sowie insbesondere zumindest eines weiteren Aufnahmeparameters automatisch erfolgt.

4. Verfahren nach einem der vorhergehenden Ansprüche,
**dadurch gekennzeichnet, dass**
das Aufnehmen (1, 13) der ersten und/oder der zweiten Röntgenaufnahme (20) ein automatisches Variieren des Aufnahmewinkels während des Aufnehmens (1, 13) um wenige Grad umfasst, insbesondere weniger als 10°, bevorzugt weniger als 5° und besonders bevorzugt weniger als 2°.

5. Verfahren nach einem der vorhergehenden Ansprüche,
**dadurch gekennzeichnet, dass**
das automatische Analysieren (9, 14) ein Detektieren zumindest eines Bereichs des Körperteils (21) mit vorgegebenen Eigenschaften, insbesondere einen Bereich eines Blutgefäßes mit einer Stenose, und/oder einer Position des Körperteils (21) und/oder einer Orientierung des Körperteils (21) und/oder zumindest einer Abmessung des Körperteils (21) und/oder eines Verlaufs des Körperteils (21) und/oder einer Rotationsachse (24), um welche die zweite Röntgenaufnahme gegenüber der ersten Röntgenaufnahme (20) für ein Erstellen (15) eines dreidimensionalen Abbildes des Körperteils (21) verkippt wird, umfasst.

6. Verfahren nach einem der vorhergehenden Ansprüche,
**dadurch gekennzeichnet, dass**
das vorgegebene Kriterium für das Bewerten (7) der Eignung eines oder mehrere der folgenden Kriterien umfasst:
- eine Winkeldifferenz zwischen dem ersten Aufnahmewinkel und dem zumindest einen weiteren Aufnahmewinkel;
- eine Kollisionswahrscheinlichkeit des Röntgengerätes (30), insbesondere der Aufnahmeeinheit (31), mit einem weiteren Gerät und/oder der Bedienperson und/oder einem Patienten (32);
- eine Dosisbelastung für den Patienten (32);
- eine Anfahrbarkeit des Aufnahmewinkels durch die Aufnahmeeinheit (31);
- eine Pfadlänge der Röntgenstrahlung in dem Patienten (32) ;
- eine gemäß einem Modell auftretende Anzahl von Überlagerungen weiterer Körperteile mit dem Körperteil (32) in der Röntgenaufnahme (20).

7. Verfahren nach einem der vorhergehenden Ansprüche,
**dadurch gekennzeichnet, dass**
die Mindestanforderung einen vorgegebenen Winkelbetrag umfasst, um welchen sich der erste und der zweite Aufnahmewinkel unterscheiden muss, welcher insbesondere 20° oder 30° oder 40° beträgt.

8. Verfahren nach einem der vorhergehenden Ansprüche,
**dadurch gekennzeichnet, dass**
das Röntgengerät (30) einen Patiententisch (34) mit einer elektrischen Verstellfunktion umfasst, wobei die Verstellfunktion während des Aufnehmens (6, 13) der ersten und/oder zweiten Röntgenaufnahme (20) deaktiviert wird, insbesondere in dem Zeitraum zwischen den beiden Röntgenaufnahmen entweder die Verstellfunktion deaktiviert wird oder ein Verstellen des Patiententisches (34) durch eine Detektiereinheit detektiert und bei dem Einstellen (11) des zweiten Aufnahmewinkels berücksichtigt wird.

9. Verfahren nach einem der vorhergehenden Ansprüche,
**gekennzeichnet durch** ein
automatisches Vorauswählen von korrelierten Einzelbildern der beiden Röntgenaufnahmen durch die Recheneinheit (33) unter Berücksichtigung von Ergebnissen des automatischen Analysierens (9, 14) und/oder des Bewertens (10) der Eignung für das Erstellen (15) des dreidimensionalen Abbilds.

10. Verfahren nach einem der vorhergehenden Ansprüche,
**dadurch gekennzeichnet, dass**
nach dem Aufnehmen (13) der zweiten Röntgenaufnahme, insbesondere nach dem Erstellen (15) des dreidimensionalen Abbildes des Körperteils (21), ein Analysieren der zweiten Röntgenaufnahme, insbesondere des dreidimensionalen Abbilds, erfolgt, sowie ein Bewerten der Eignung von zumindest einem weiteren Aufnahmewinkel für das Erstellen eines verbesserten dreidimensionalen Abbilds des Körperteils (21), durch die Recheneinheit (33) unter Berücksichtigung eines Ergebnisses des automatischen Analysierens, wobei für das Erstellen des verbesserten dreidimensionalen Abbildes eine dritte Röntgenaufnahme aus einem dritten Aufnahmewinkel vorgesehen ist, und ein analoges Durchführen der für die zweite Röntgenaufnahme bekannten Schritte des Einstellens (11), des manuell kontrollierten Anfahrens (12) sowie des Aufnehmens (13) für die dritte Röntgenaufnahme.

11. Röntgengerät (30) zum Aufnehmen von zumindest zwei Röntgenbildern für ein Erstellen (15) eines dreidimensionalen Abbilds eines Körperteils (21), mit einer Aufnahmeeinheit (31) zum Aufnehmen (6, 13) von Röntgenaufnahmen aus an dem Röntgengerät (30) einstellbaren und durch die Aufnahmeeinheit (31) unter manueller Kontrolle anfahrbaren Aufnahmewinkeln, wobei das Röntgengerät (30) auch eine Recheneinheit (33) aufweist zum automatischen Analysieren (9, 14) von zumindest einer an die Recheneinheit (33) bereitgestellten ersten Röntgenaufnahme, welche aus einem ersten Aufnahmewinkel aufgenommen ist, und zum Bewerten (10) einer Eignung von zumindest einem weiteren Aufnahmewinkel für das Erstellen (15) des dreidimensionalen Abbilds des Körperteils (32) unter Berücksichtigung eines Ergebnisses des automatischen Analysierens (9, 14), wobei für das Erstellen (15) des dreidimensionalen Abbildes eine zweite Röntgenaufnahme (20) aus einem zweiten Aufnahmewinkel durch die Aufnahmeeinheit (31) aufnehmbar ist,
**dadurch gekennzeichnet, dass**
die Recheneinheit dazu eingerichtet ist, bei dem Bewerten der Eignung für ein vorgegebenes Kriterium zumindest eine Mindestanforderung zu berücksichtigen und bei einem Nichterfüllen der Mindestanforderung durch den bewerteten Aufnahmewinkel das Einstellen (11) von dem bewerteten Aufnahmewinkel als zweiten Aufnahmewinkel zu verhindern und/oder eine Bedienperson bei einem Einstellen (11) von dem bewerteten Aufnahmewinkel als zweitem Aufnahmewinkel zu warnen.

## Claims

1. Method for the operation of an X-ray machine (30) for the generation (15) of a three-dimensional reconstruction of a body part (21), having the steps:
- supplying (7) a first X-ray capture (20) of the body part (21), which was captured from a first capture angle, to a computing unit (33) of the X-ray machine (30);
- automatic analysis (9) of the supplied first X-ray capture (20) by the computing unit (33);
- evaluation (10) of the suitability of at least one further capture angle for the generation (15) of the three-dimensional reconstruction of the body part (21) by the computing unit (33) in the light of a result from the automatic analysis (9), a second X-ray capture from a second capture angle being captured for the generation (15) of the three-dimensional reconstruction;
- setting (11) of the second capture angle on the X-ray machine (30), either automatically by the computing unit (33), a result from the evaluation (10) being taken into account, or manually by an operator, a result from the evaluation (10) being displayed to the operator by a display unit (35) of the X-ray machine (30);
- manually controlled approach (12) to the set second capture angle by a capture unit (31) of the X-ray machine (30) ;
- capture (13) of the second X-ray capture (20) from the approached second capture angle by the capture unit (31) **characterised in that**
- the evaluation (7) of suitability for a specified criterion takes account of at least one minimum requirement and, in the event of the minimum requirement not being met by the evaluated capture angle, the computing unit (33) prevents the evaluated capture angle from being set (11) as the second capture angle and/or warns the operator in the event of the evaluated capture angle being set (11) as the second capture angle.

2. Method according to claim 1,
**characterised by**
- selection (1) by the operator of "generation of a three-dimensional reconstruction" as the intended purpose for the first X-ray capture (20) from a plurality of possible intended purposes for captures of the X-ray machine (30);
- automatic setting (3) of at least one capture parameter for the first X-ray capture (20);
- setting (4) of the first capture angle;
- manually controlled approach (5) to the set first capture angle by a capture unit (31) of the X-ray machine (30) ;
- capture (6) of the first X-ray capture (20) from the approached first capture angle by the capture unit (31).

3. Method according to claim 2,
**characterised in that**
- selection (1) also includes specifying (2) the body part (32) to be reconstructed and
- setting (4) of the first capture angle and in particular of at least one further capture parameter proceeds automatically.

4. Method according to one of the preceding claims,
**characterised in that**
capture (1, 13) of the first and/or second X-ray capture (20) includes automatic variation of the capture angle during capture (1, 13) by a few degrees, in particular less than 10°, preferably less than 5° and particularly preferably less than 2°.

5. Method according to one of the preceding claims,
**characterised in that**
the automatic analysis (9, 14) includes detection of at least one region of the body part (21) with specified characteristics, in particular a region of a blood vessel with a stenosis, and/or a position of the body part (21) and/or an orientation of the body part (21) and/or at least one dimension of the body part (21) and/or a course of the body part (21) and/or an axis of rotation (24), about which the second X-ray capture is tilted relative to the first X-ray capture (20) for the generation (15) of a three-dimensional reconstruction of the body part (21).

6. Method according to one of the preceding claims,
**characterised in that**
the specified criterion for the evaluation (7) of suitability includes one or more of the following criteria:
- an angular difference between the first capture angle and the at least one further capture angle;
- a probability of the X-ray machine (30), in particular the capture unit (31), colliding with a further item of equipment and/or the operator and/or a patient (32);
- a dose burden for the patient (32);
- approachability of the capture angle by the capture unit (31) ;
- a path length of the X-rays in the patient (32);
- a number of further body parts superposed over the body part (32) in the X-ray capture (20) in accordance with a model.

7. Method according to one of the preceding claims,
**characterised in that**
the minimum requirement includes a specified angular amount by which the first and second capture angles must differ, said amount in particular amounting to 20° or 30° or 40°.

8. Method according to one of the preceding claims,
**characterised in that**
the X-ray machine (30) comprises a patient table (34) with an electrical adjustment function, the adjustment function being disabled during capture (6, 13) of the first and/or second X-ray capture (20), in particular in the period between the two X-ray captures the adjustment function either being disabled or adjustment of the patient table (34) being detected by a detection unit and taken into account during setting (11) of the second capture angle.

9. Method according to one of the preceding claims,
**characterised by**
automatic preselection of correlated frames of the two X-ray captures by the computing unit (33) in the light of results of the automatic analysis (9, 14) and/or of the evaluation (10) of suitability for the generation (15) of the three-dimensional reconstruction.

10. Method according to one of the preceding claims,
**characterised in that**
once the second X-ray capture has been captured (13), in particular once the three-dimensional reconstruction of the body part (21) has been generated (15), an analysis of the second X-ray capture, in particular of the three-dimensional reconstruction, is carried out, as is an evaluation of the suitability of at least one further capture angle for the generation of an improved three-dimensional reconstruction of the body part (21), by the computing unit (33) in the light of a result from the automatic analysis, a third X-ray capture from a third capture angle being provided for the generation of the improved three-dimensional reconstruction, and the steps of setting (11), manually controlled approach (12) and capture (13) known for the second X-ray capture being carried out similarly for the third X-ray capture.

11. X-ray machine (30) for the capture of at least two X-ray images for the generation (15) of a three-dimensional reconstruction of a body part (21) with a capture unit (31) for the capture (6, 13) of X-ray captures from capture angles settable on the X-ray machine (30) and approachable by the capture unit (31) under manual control, wherein the X-ray machine (30) also has a computing unit (33) for the automatic analysis (9, 14) of at least one first X-ray capture supplied to the computing unit (33), which first X-ray capture is captured from a first capture angle, and for the evaluation (10) of the suitability of at least one further capture angle for the generation (15) of the three-dimensional reconstruction of the body part (32) in the light of a result from the automatic analysis (9, 14), it being possible for the capture unit (31) to capture a second X-ray capture (20) from a second capture angle for the generation (15) of the three-dimensional reconstruction,
**characterised in that**
the computing unit is configured, during the evaluation (7) of suitability for a specified criterion, to take account of at least one minimum requirement and, in the event of the minimum requirement not being met by the evaluated capture angle, to prevent the evaluated capture angle from being set (11) as the second capture angle and/or to warn an operator in the event of the evaluated capture angle being set (11) as the second capture angle.

## Revendications

1. Procédé pour faire fonctionner un appareil (30) à rayons X pour la production (15) d'une image tridimensionnelle d'une partie (21) du corps, comprenant les stades :
- mise à disposition (7) d'une unité (33) informatique de l'appareil (30) à rayons X d'une première radiographie (20) de la partie (21) du corps, qui a été prise à partir d'un premier angle de prise de vues;
- analyse (9) automatique, par l'unité (33) informatique, de la première radiographie (20) mise à disposition;
- évaluation (10), par l'unité (33) informatique, de l'aptitude d'au moins au autre angle de prise de vues à la production (15) de l'image tridimensionnelle de la partie (21) du corps, en tenant compte d'un résultat de l'analyse (9) automatique, dans lequel, pour la production (15) de l'image tridimensionnelle, on prend une deuxième radiographie à partir d'un deuxième angle de prise de vues;
- mise (11) du deuxième angle de prise de vues sur l'appareil (30) à rayons X, soit automatiquement par l'unité (33) informatique, un résultat de l'évaluation (10) étant pris en compte, soit manuellement par un opérateur, un résultat de l'évaluation (10) étant indiqué à l'opérateur par une unité (35) d'affichage de l'appareil (30) à rayons X.
- approche (12) contrôlée manuellement du deuxième angle de prise de vues, qui a été mis, par une unité (31) de prise de vues de l'appareil (30) à rayons X;
- prise (13) de la deuxième radiographie (20) sous le deuxième angle de prise de vues approché par l'unité (31) de prise de vues,
**caractérisé en ce que**
- l'évaluation (7) de l'aptitude, pour un critère donné à l'avance, prend en compte au moins une exigence minimum et, au moyen de l'unité (33) informatique, est empêchée, si l'exigence minimum n'est pas satisfaite par l'angle de prise de vues évalué, la mise de l'angle de prise de vues évalué comme deuxième angle de prise de vues et/ou l'opérateur est averti, lors de la mise (11), de l'angle de prise de vues évalué comme deuxième angle de prise de vues.

2. Procédé suivant la revendication 1,
**caractérisé par** un
- choix (1) par l'opérateur de la "production d'une image tridimensionnelle" parmi une pluralité de buts de détermination possibles pour des prises de vue de l'appareil (30) à rayons X comme buts de détermination pour la première radiographie (20);
- réglage (3) automatique d'au moins un paramètre de prise de vues pour la première radiographie (20);
- mise (4) du premier angle de prise de vues;
- approche (5) contrôlée manuellement, du premier angle de prise de vues approché, par l'unité (31) de prise de vues de l'appareil (30) à rayons X;
- prise (6) de la première radiographie (20) par l'unité (31) de prise de vues sous le premier angle de prise de vues approché.

3. Procédé suivant la revendication 2,
**caractérisé en ce que**
- le choix (1) comprend aussi une précision (2) de la partie (32) du corps à reproduire et
- la mise (4) du premier angle de prise de vues, ainsi que notamment le réglage d'au moins un autre paramètre de prise de vues, s'effectue automatiquement.

4. Procédé suivant l'une des revendications précédentes,
**caractérisé en ce que**
la prise (1, 13) de la première et/ou de la deuxième radiographie (20) comprend une variation automatique de l'angle de prise de vues pendant la prise (1, 13) de quelques degrés, notamment de moins de 10°, de préférence de moins de 5° et d'une manière particulièrement préférée,
de moins de 2°.

5. Procédé suivant l'une des revendications précédentes,
**caractérisé en ce que**
l'analyse (9, 14) automatique comprend la détection d'au moins une région de la partie (21) du corps ayant des propriétés données à l'avance, notamment d'une région d'un vaisseau sanguin ayant une sténose et/ou d'une position de la partie (21) du corps et/ou d'une orientation de la partie (21) du corps et/ou d'au moins une dimension de la partie (21) du corps et/ou d'un tracé de la partie (21) du corps et/ou d'un axe (24) de rotation autour duquel la deuxième radiographie est basculée par rapport à la première radiographie (20) pour la production (15) d'une image tridimensionnelle de la partie (21) du corps.

6. Procédé suivant l'une des revendications précédentes,
**caractérisé en ce que**
le critère donné à l'avance d'évaluation (7) de l'aptitude comprend un ou plusieurs des critères suivants :
- une différence angulaire entre le premier angle de prise de vues et le au moins un autre angle de prise de vues;
- une probabilité de collision de l'appareil (30) à rayons X, notamment de l'unité (31) de prise de vues, avec un autre appareil et/ou l'opérateur et/ou un patient (32);
- une charge de dose du patient (32);
- une possibilité d'approche de l'angle de prise de vues par l'unité (31) de prise de vues;
- une longueur de trajet du rayonnement X dans le patient (32);
- un nombre, se produisant suivant un modèle, de superpositions d'autres parties du corps avec la partie (32) du corps dans la radiographie (20).

7. Procédé suivant l'une des revendications précédentes,
**caractérisé en ce que**
l'exigence minimum comprend une valeur angulaire absolue données à l'avance, dont le premier et le deuxième angles de prise de vues doivent différer, laquelle est notamment de 20° ou de 30° ou de 40°.

8. Procédé suivant l'une des revendications précédentes,
**caractérisé en ce que**
l'appareil (30) à rayon X comprend une couchette (34) de patient ayant une fonction de réglage électrique, la fonction de réglage étant désactivée pendant la prise (6, 13) de la première et/ou de la deuxième radiographie (20), notamment dans le laps de temps compris entre les deux radiographies ou bien la fonction de réglage est désactivée ou un réglage de la couchette (34) du patient, par une unité de détection, est détecté et il en est tenu compte lors de la mise (11) du deuxième angle de prise de vues.

9. Procédé suivant l'une des revendications précédentes,
**caractérisé par** un
choix préalable automatique d'images individuelles corrélées des deux radiographies par l'unité (33) informatique, en tenant compte d'un résultat de l'analyse (9, 14) automatique et/ou de l'évaluation (10) de l'aptitude à la production (15) de l'image tridimensionnelle.

10. Procédé suivant l'une des revendications précédentes,
**caractérisé en ce que**
après la prise (13) de la deuxième radiographie, notamment après la production (15) de l'image tridimensionnelle de la partie (21) du corps, une analyse de la deuxième radiographie, notamment de l'image tridimensionnelle, est effectuée, ainsi qu'une évaluation de l'aptitude d'au moins un autre angle de prise de vue à la production d'une image tridimensionnelle améliorée de la partie (21) du corps par l'unité (33) informatique, en tenant compte d'un résultat de l'analyse automatique, dans lequel, pour produire l'image tridimensionnelle améliorée, il est prévu une troisième radiographie à partir d'un troisième angle de prise de vues et d'effectuer, de manière analogue, les stades connus pour la deuxième radiographie de mise (11) de l'angle, de l'approche (12), contrôlée de manière manuelle, ainsi que de prise (13) de la troisième radiographie.

11. Appareil (30) à rayons X pour prendre au moins deux radiographies, afin de produire (15) une image tridimensionnelle d'une partie (21) du corps, comprenant une unité (31) de prise de vues pour prendre (6, 13) des radiographies, à partir d'angles de prise vues réglables sur l'appareil (30) à rayons X et dont on peut s'approcher, sous contrôle manuel, par l'unité (31) de prise de vues, l'appareil (30) à rayons X ayant aussi une unité (33) informatique pour analyser (9, 14) automatiquement au moins une première radiographie, qui est mise à disposition de l'unité (33) informatique et qui a été prise à partir d'un premier angle de prise de vues, et pour évaluer (10) l'aptitude d'au moins un autre angle de prise de vues à la production (15) de l'image tridimensionnelle de la partie (32) du corps, en tenant compte d'un résultat de l'analyse (9, 14) automatique, dans lequel, pour produire l'image tridimensionnelle, une deuxième radiographie (20), à partir d'un deuxième angle de prise de vue, peut être prise par l'unité (31) de prise de vues,
**caractérisé en ce que**
l'unité informatique est conçue pour, lors de l'évaluation de l'aptitude pour un critère donné à l'avance, tenir compte au moins d'une exigence minimum et, lorsque l'exigence minimum n'est pas satisfaite, pour empêcher, par l'angle de prise de vues évalué, la mise (11) de l'angle de prise de vues évalué, comme deuxième angle de prise de vue et/ou pour avertir un opérateur, lors de la mise (11) de l'angle de prise de vues évalué comme deuxième angle de prise de vues.
